# EUROPEAN PATENT SPECIFICATION

(11) **EP 3 659 539 B1**
(45) Date of publication and mention of the grant of the patent: **13.04.2022**
(21) Application number: 20153886.5
(22) Date of filing: 07.12.2017
(51) Int. Cl.: A61B 18/14, A61B 18/00, A61B 34/20

(54) **IRRIGATED BALLOON CATHETER WITH SUPPORT SPINES AND VARIABLE SHAPE**
GESPÜLTER BALLONKATHETER MIT STÜTZDORNEN UND VARIABLER FORM
CATHÉTER À BALLONNET IRRIGUÉ Á COLONNES DE SUPPORT ET FORME VARIABLE

(30) Priority: 08.12.2016 US 201662431773 P; 30.11.2017 US 201715827111
(43) Date of publication of application: 03.06.2020
(62) Divisional of application: 17205876.0
(73) Proprietor: Biosense Webster (Israel), Ltd., 2066717 Yoknaem (IL)
(72) Inventor: BASU, Shubhaya, Irwindale, CA 91706 (US); FUENTES-ORTEGA, Cesar, Irwindale, CA 91706 (US)
(74) Representative: Carpmaels & Ransford LLP

(56) References cited:
- US-A- 6 123 718
- US-A1- 2011 301 587
- US-A1- 2014 357 956
- US-A1- 2016 183 877

## Description

### FIELD

This disclosure relates to medical devices. More particularly, this disclosure relates to improvements in cardiac catheterization, including electrophysiologic (EP) catheters, in particular, EP catheters for mapping and/or ablating regions in the heart, including the atrium, an ostium and tubular regions in the heart.

### BACKGROUND

Cardiac arrhythmias, such as atrial fibrillation, occur when regions of cardiac tissue abnormally conduct electric signals to adjacent tissue, thereby disrupting the normal cardiac cycle and causing asynchronous rhythm.

Procedures for treating arrhythmia include surgically disrupting the origin of the signals causing the arrhythmia, as well as disrupting the conducting pathway for such signals. By selectively ablating cardiac tissue by application of energy via a catheter, it is sometimes possible to cease or modify the propagation of unwanted electrical signals from one portion of the heart to another. The ablation process destroys the unwanted electrical pathways by formation of non-conducting lesions.

Circumferential lesions at or near the ostia of the pulmonary veins have been created to treat atrial arrhythmias. U.S. Pat. Nos. 6,012,457 and 6,024,740, both to Lesh, disclose a radially expandable ablation device, which includes a radiofrequency electrode. Using this device, it is proposed to deliver radiofrequency energy to the pulmonary veins in order to establish a circumferential conduction block, thereby electrically isolating the pulmonary veins from the left atrium.

U.S. Pat. No. 6,814,733 to Schwartz et al. describes a catheter introduction apparatus having a radially expandable helical coil as a radiofrequency emitter. In one application the emitter is introduced percutaneously, and trans-septally advanced to the ostium of a pulmonary vein. The emitter is radially expanded, which can be accomplished by inflating an anchoring balloon about which the emitter is wrapped, in order to cause the emitter to make circumferential contact with the inner wall of the pulmonary vein. The coil is energized by a radiofrequency generator, and a circumferential ablation lesion is produced in the myocardial sleeve of the pulmonary vein, which effectively blocks electrical propagation between the pulmonary vein and the left atrium.

Another example is found in U.S. Pat. No. 7,340,307 to Maguire, et al., which proposes a tissue ablation system and method that treats atrial arrhythmia by ablating a circumferential region of tissue at a location where a pulmonary vein extends from an atrium. The system includes a circumferential ablation member with an ablation element and includes a delivery assembly for delivering the ablation member to the location. The circumferential ablation member is generally adjustable between different configurations to allow both the delivery through a delivery sheath into the atrium and the ablative coupling between the ablation element and the circumferential region of tissue.

More recently, inflatable catheter electrode assemblies have been constructed with flex circuits to provide the outer surface of the inflatable electrode assemblies with a multitude of very small electrodes. Examples of catheter balloon structures are described in U.S. Publication No. 2016/0175041, titled Balloon for Ablation Around Pulmonary Vein.

Flex circuits or flexible electronics involve a technology for assembling electronic circuits by mounting electronic devices on flexible plastic substrates, such as polyimide, Liquid Crystal Polymer (LCP), PEEK or transparent conductive polyester film (PET). Additionally, flex circuits can be screen printed silver circuits on polyester. Flexible printed circuits (FPC) are made with a photolithographic technology. An alternative way of making flexible foil circuits or flexible flat cables (FFCs) is laminating very thin (0.07 mm) copper strips in between two layers of PET. These PET layers, typically 0.05 mm thick, are coated with an adhesive which is thermosetting, and will be activated during the lamination process. Single-sided flexible circuits have a single conductor layer made of either a metal or conductive (metal filled) polymer on a flexible dielectric film. Component termination features are accessible only from one side. Holes may be formed in the base film to allow component leads to pass through for interconnection, normally by soldering.

However, due to variances in human anatomy, ostia and tubular regions in the heart come in all sizes. Thus, conventional balloon or inflatable catheters may not have the necessary flexibility to accommodate different shapes and sizes while having sufficient structural support for effective circumferential contact with tissue. Moreover, the balloon may tend to buckle or bend off-axis when the balloon comes into contact with tissue.

Accordingly, there is a desire for a balloon or a catheter having an inflatable balloon that can more reliably maintain its overall spherical shape yet be variable in its length and radius by selective manipulation of a user.

US 2014/357956 A1 describes cardiac ablation catheters and methods of use. The catheter includes at least one camera inside an expandable membrane for visualizing an ablation procedure.

### SUMMARY

The invention is defined in the appended claims. The present disclosure is directed to a catheter having an irrigated inflatable balloon adapted for use in regions of the heart, including, for example, the atrium, ostia and pulmonary veins. The balloon includes contact electrodes on its membrane, wherein a user may vary the balloon's configuration by manipulating an elongated expander that extends along the length of the catheter and through the balloon's interior, with its distal end coupled to a distal end of the balloon. The expander may pass through an irrigation lumen to save on space within the catheter. Moreover, the expander itself may be hollow in providing a lumen through which components, such as cables or lead wires, can pass between the balloon and a control handle. One or more segments of the expander may include flexure slits for increased flexibility along its length. The distal end of the balloon includes a housing for components, including a position sensor. Notwithstanding the housing, the balloon's distal end and the manner by which the balloon membrane is attached to the housing present a generally flat atraumatic surface suitable for direct head-on contact with tissue in the atrium.

To support the shape of the balloon, and help the balloon remain on-axis relative to the catheter shaft during tissue contact, the balloon includes support spines that span longitudinally from a proximal end of the balloon toward the distal end of the balloon. The spines may be evenly spaced around the balloon and the length of the spines may span the entire length of the balloon, or a portion thereof, as needed or desired.

The spines may extend through a passage provided by a protective cover or sleeve that is affixed to the balloon membrane. The passage may receive and protect other components extending along an outer surface of the balloon.

In some embodiments, an electrophysiology catheter includes an elongated catheter shaft having a first lumen and a balloon having a membrane supporting a contact electrode. The catheter also includes an irrigation tubing and an elongated expander, wherein the irrigation tubing extends through the catheter shaft and the expander extends through a lumen of the irrigation tubing. The irrigation tubing terminates at a proximal end of the balloon, whereas the expander extends into the balloon and is coupled to a distal end of the balloon at its distal end. The expander is advantageously longitudinally movable relative to the catheter shaft to move the distal end of the balloon in changing a configuration of the balloon.

The electrophysiology catheter includes a plurality of support spines extending longitudinally along an outer surface of the membrane of the balloon. Some spines may extend from the proximal end of the balloon to the distal end of the balloon and/or some spines may extend from the proximal end of the balloon to a location proximal of the distal end of the balloon. In some detailed embodiments, one or more support spines extend from the distal end of the balloon to a location distal of the proximal end of the balloon. The balloon may include protective covers for the spines. The covers may be in the form of strips or sleeves affixed to the balloon membrane or to proximal tail portions of a flex circuit electrode assembly providing the contact electrode.

In some detailed embodiments, the distal end of the balloon includes a housing having a flat distal face, and an outer radial surface to which an inwardly turned distal end portion of the balloon membrane is affixed, in providing the distal end of the balloon with an atraumatic profile.

In some detailed embodiments, the expander is hollow, having a lumen configured to receive components, including, for example, cables and/or lead wires. In some embodiments, the expander has a segment with one or more intermittent cuts or spiral slits for increased flexibility.

In other embodiments, an electrophysiology catheter includes an elongated catheter shaft having a first lumen, and a balloon having a membrane and a flex circuit electrode assembly, the balloon also having a distal housing for a component with an electrical conduit. The catheter also includes a hollow elongated expander longitudinally movable through the first lumen relative to the catheter shaft, the expander having a second lumen through which the electrical conduit passes, and a distal end coupled to the distal housing for changing a shape of the balloon.

In some detailed embodiments, the balloon of the catheter includes a support spine extending longitudinally along the balloon membrane. In some detailed embodiments, the support spine extends from the proximal end of the balloon to the distal end of the balloon. In some detailed embodiments, the support spine extends from the proximal end of the balloon to a location proximal of the distal end of the balloon. In some detailed embodiments, the support spine extends from the distal end of the balloon to a location distal of the proximal end of the balloon.

In some detailed embodiments, the balloon has an atraumatic distal end. In some detailed embodiments, the distal end of the balloon includes a flat distal face, and a distal end portion the balloon membrane is turned inwardly and affixed to the distal end of the housing.

In some embodiments, lead wires for the flex circuit electrode assembly extend along the membrane outside of the interior of the balloon, from a proximal end of the flex circuit electrode to the proximal end of the balloon. Alternatively, the lead wires for the flex circuit electrode assembly can extend through the expander lumen, exit the distal end of the balloon and connect to distal ends of the flex circuit electrodes.

### BRIEF DESCRIPTION OF THE DRAWINGS

These and other features and advantages of the present disclosure will be better understood by reference to the following detailed description when considered in conjunction with the accompanying drawings. It is understood that selected structures and features have not been shown in certain drawings so as to provide better viewing of the remaining structures and features.
FIG. 1 is a schematic illustration of an invasive medical procedure.
FIG. 2A is a top plan view of a balloon catheter of the present disclosure in its inflated state, according to an embodiment of the present disclosure.
FIG. 2B is an end cross-sectional view of an intermediate section of the catheter of FIG. 2A, taken along line A-A.
FIG. 3 is a front perspective view of a balloon of the balloon catheter, according to an embodiment of the present disclosure.
FIG. 4 is a side view of the balloon deployed in the region of a pulmonary vein and its ostium.
FIG. 5 is a top plan view of a plurality of flex circuit electrode assembly.
FIG. 6A is a rear perspective view of the balloon of FIG. 3.
FIG. 6B is an alternative embodiment of FIG. 6A.
FIG. 7 is a flex circuit electrode assembly, according to an embodiment of the present disclosure, partially lifted from the balloon.
FIG. 8 is a top plan view of a flex circuit electrode assembly.
FIG. 9 is a side cross-sectional view of the catheter of FIG. 2A, including a proximal end of the balloon, taken along line B-B.
FIG. 10 is a side cross-sectional view of a distal end of the balloon, according to an embodiment of the present disclosure.
FIG. 11 is a side view of an expander with flexure slits, with a heat shrink sleeve shown partially broken away, according to an embodiment of the present disclosure.
FIG. 12 is an end cross-section view of the proximal end of FIG. 9.
FIG. 13 is an end cross-sectional view of a proximal tail and a support spine with its cover, according to an embodiment of the present disclosure.
FIG. 14 is a front perspective view of a balloon of the balloon catheter, according to another embodiment of the present disclosure.
FIG. 15 is a rear perspective view of the balloon of FIG. 14.

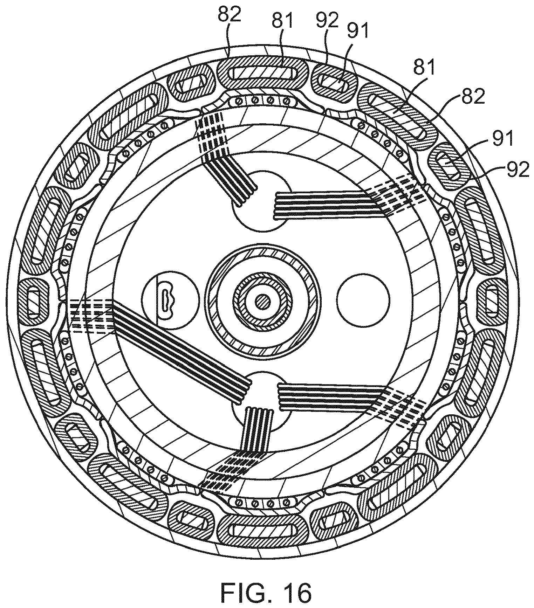

FIG. 16 is an end cross-sectional view of a proximal end of the balloon of FIG. 15.

### DETAILED DESCRIPTION

### Overview

Ablation of cardiac tissue to correct a malfunctioning heart is a well-known procedure for implementing such a correction. Typically, in order to successfully ablate, cardia electropotentials need to be measured at various locations of the myocardium. In addition, temperature measurements during ablation provide data enabling the efficacy of the ablation to be measured. Typically, for an ablation procedure, the electropotentials and the temperatures are measured before, during, and after the actual ablation.

In contrast with prior art systems that use two or more separate instructions (e.g., one for the electropotential and temperature measurements, and another for the ablation), embodiments of the present disclosure facilitate the two measurements, and in addition enable ablation using radiofrequency electromagnetic energy, using a single balloon catheter. The catheter has a lumen, and an inflatable balloon is deployed through the catheter lumen (the balloon travels through the lumen in a collapsed, deflated configuration, and the balloon is inflated on exiting the lumen). The balloon has an exterior wall or membrane and has a distal end and a proximal end which define a longitudinal axis that extends the lumen.

The catheter includes an elongated expander which is longitudinally movable relative to a catheter shaft for lengthening or compressing the balloon to alter its shape. The expander has a length that extends from the control handle, through the catheter shaft, through a proximal end of the balloon and into the interior of the balloon to a distal end of the balloon. The distal end of the balloon is coupled to a distal end of the expander whose longitudinal movement extends distally or withdraws proximally the distal end of the balloon in lengthening or compressing the balloon. The expander may pass through the lumen of an irrigation tubing supplying irrigation fluid to the balloon, such that the expander and the irrigation fluid share a common lumen as an efficient use of space within the catheter.

The balloon also includes support spines that are positioned on the balloon membrane spread radially around the balloon. Selected support spines may extend longitudinally from the proximal end of the balloon partially to the distal end, e.g., to an equatorial region of the balloon. Other support spines, in addition to or in lieu of the selected spines, may extend longitudinally from the proximal end of the balloon to the distal end. Alternatively, the support spines can extend from the distal end of the balloon partially to the proximal end, e.g. to an equatorial region of the balloon, distal to the proximal end of the balloon. Optionally, the support spines can be hollow and the lumens thereof can be used to run lead wires for the electrodes from a proximal portion of the balloon to the electrodes.

A multi-layer flexible electrode assembly is attached to an exterior wall or membrane of the balloon. The structure comprises a plurality of electrode groups arranged circumferentially about the longitudinal axis, where each electrode group comprises multiple contact and wiring electrodes arranged longitudinally. One or more electrode group may also include at least one micro-electrode that is insulated physically and electrically from the electrodes in its group. Each electrode group may also include at least a thermocouple.

Using a single balloon catheter, with at least the three functionalities of ability to perform ablation, electropotential measurement, and temperature measurement, simplifies cardiac ablation procedures.

### System Description

In the following description, like elements in the drawings are identified by like numerals, and like elements are differentiated as necessary by appending a letter to the identifying numeral.

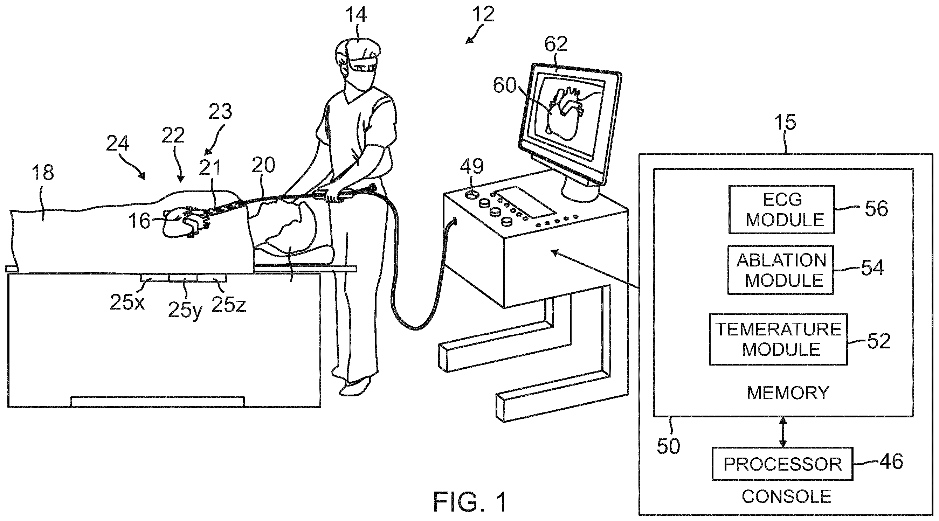

**FIG. 1** is a schematic illustration of an invasive medical procedure using apparatus 12, according to an embodiment of the present disclosure. The procedure is performed by a medical professional 14, and, by way of example, the procedure in the description hereinbelow is assumed to comprise ablation of a portion of a myocardium 16 of the heart of a human patient 18. However, it is understood that embodiments of the present disclosure are not merely applicable to this specific procedure, and may include substantially any procedure on biological tissue or on non-biological materials.

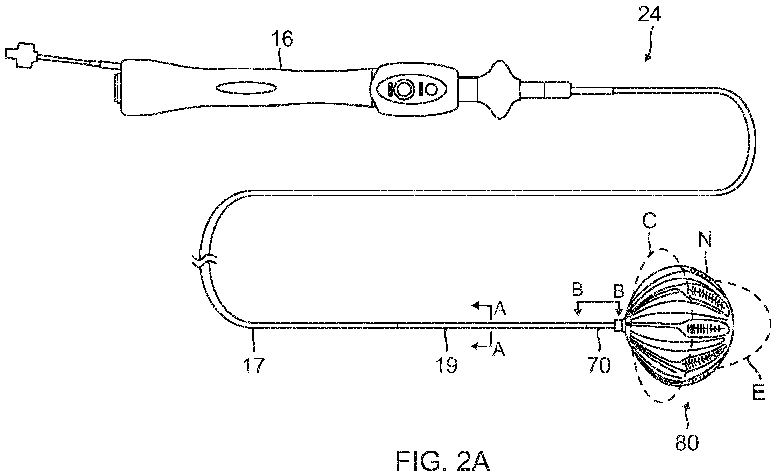

In order to perform the ablation, medical professional 14 inserts a probe 20 into a sheath 21 that has been pre-positioned in a lumen of the patient. Sheath 21 is positioned so that a distal end 22 of probe 20 enters the heart of the patient. A balloon catheter 24, which is described in more detail below with reference to **FIG. 2A****,** is deployed through a lumen 23 of the probe 20, and exits from a distal end of the probe 20.

As shown in **FIG. 1**, apparatus 12 is controlled by a system processor 46, which is located in an operating console 15 of the apparatus. Console 15 comprises controls 49 which are used by professional 14 to communicate with the processor. During the procedure, the processor 46 typically tracks a location and an orientation of the distal end 22 of the probe 20, using any method known in the art. For example, processor 46 may use a magnetic tracking method, wherein magnetic transmitters 25x, 25y and 25z external to the patient 18 generate signals in coils positioned in the distal end of the probe 20. The CARTO^{®} available from Biosense Webster, Inc. of Diamond Bar, California, uses such a tracking method.

The software for the processor 46 may be downloaded to the processor in electronic form, over a network, for example. Alternatively or additionally, the software may be provided on non-transitory tangible media, such as optical, magnetic, or electronic storage media. The tracking of the distal end 22 is typically displayed on a three-dimensional representation 60 of the heart of the patient 18 on a screen 62.

In order to operate apparatus 12, the processor 46 communicates with a memory 50, which has a number of modules used by the processor to operate the apparatus. Thus, the memory 50 comprises a temperature module 52, an ablation module 54, and an electrocardiograph (ECG) module 56, the functions of which are described below. The memory 50 typically comprises other modules, such as a force module for measuring the force on the distal end 22, a tracking module for operating the tracking method used by the processor 46, and an irrigation module allowing the processor to control irrigation provided for the distal end 22. For simplicity, such other modules are not illustrated in **FIG. 1**. The modules may comprise hardware as well as software elements.

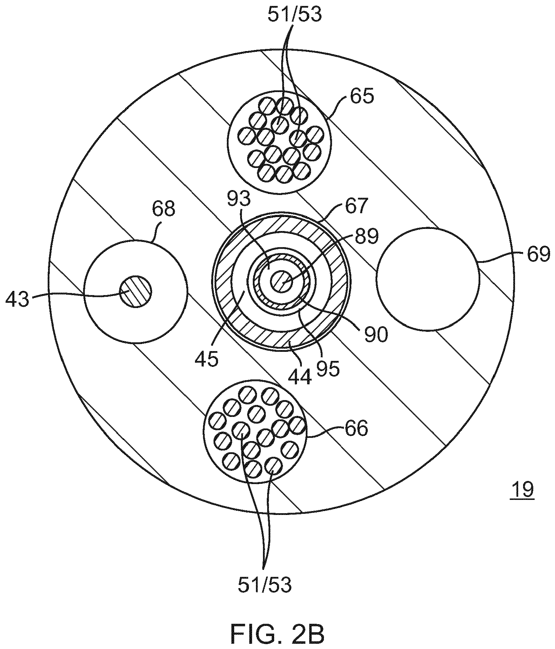

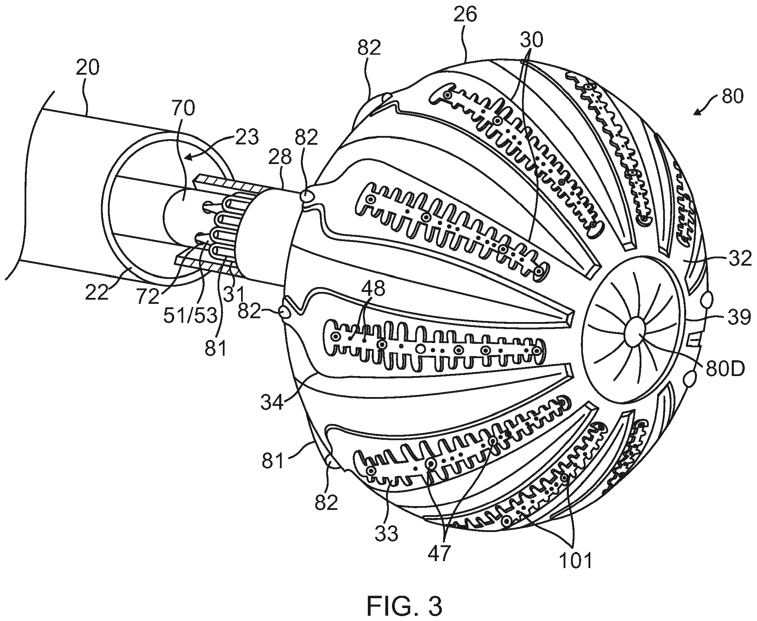

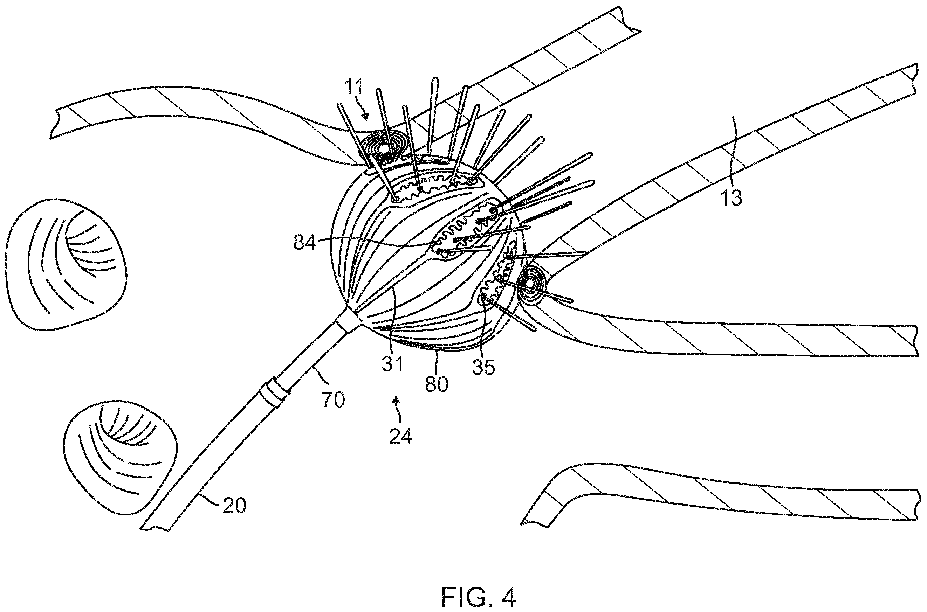

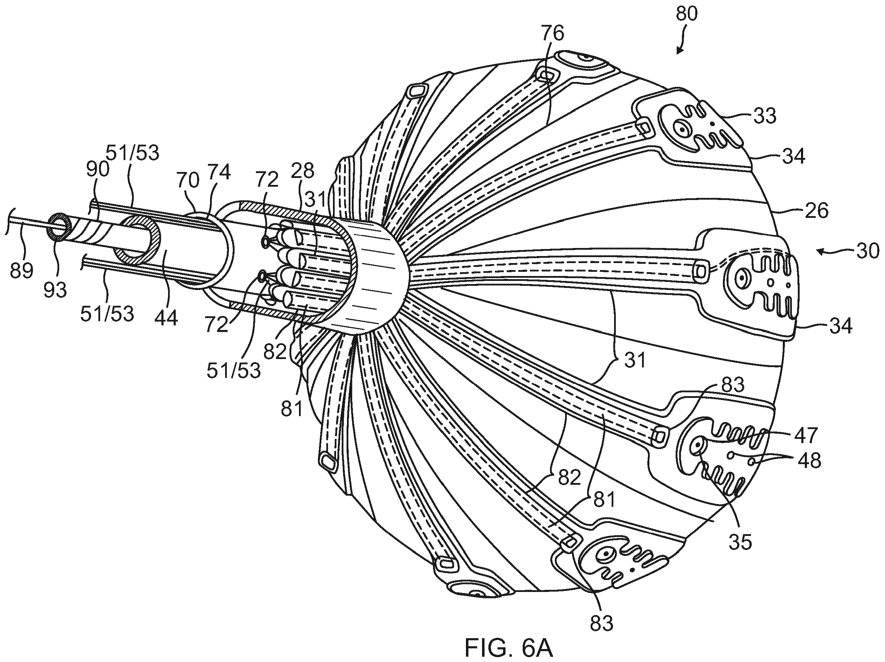

**FIG.** 3 is a schematic perspective view of a balloon 80 of the catheter 24 in its inflated configuration, according to an embodiment of the present disclosure. In a disclosed embodiment, where the balloon 80 is used to ablate an ostium 11 of a lumen, such as a pulmonary vein 13, as shown in **FIG. 4****,** the balloon 80 extends at the distal end of the catheter 24. As shown in **FIG. 2A****,** the catheter 24 has an elongated catheter shaft which may include an elongated catheter body 17, a deflectable intermediate section 19, and a tubular connector shaft 70. In some embodiments, the catheter body 17 has a central lumen, the intermediate section 19 has multiple lumens 65, 66, 67, 68 and 69 (see **FIG. 2B****),** and the shaft 70 has a central lumen 74 (see **FIG. 6A**).

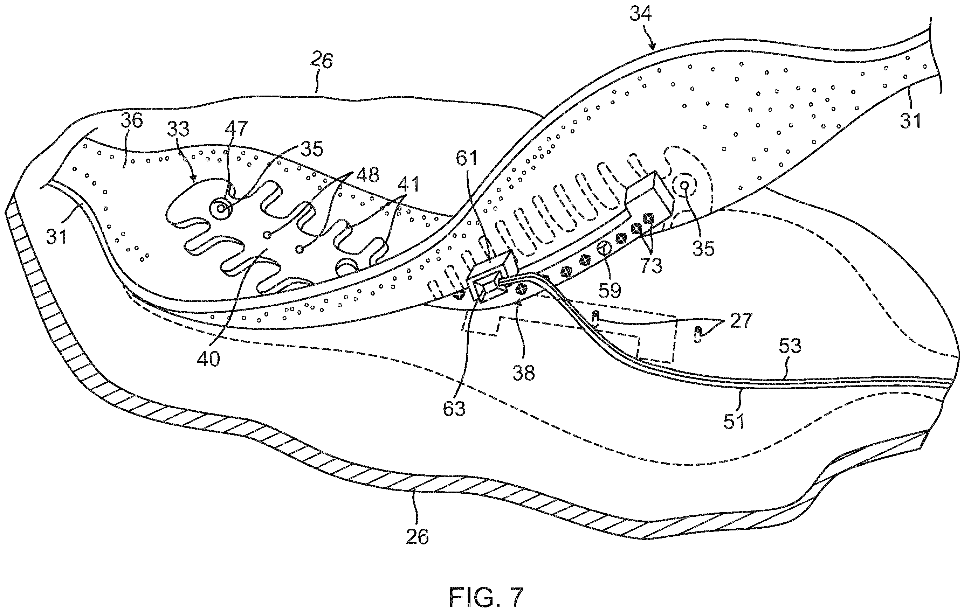

As shown in **FIG. 3****,** the inflatable balloon 80 has an exterior wall or membrane 26 of a bio-compatible material, for example, formed from a plastic such as polyethylene terephthalate (PET), polyurethane or PEBAX^{®}. The shaft 70 and a distal end 80D of the balloon 80 define a longitudinal axis. The balloon 80 is deployed, in a collapsed uninflated configuration, via the lumen 23 of the probe 20, and may be inflated after exiting from the distal end 22. The balloon 80 may be inflated and deflated by injection and expulsion of a fluid such as saline solution through the catheter shaft. The membrane 26 of the balloon 80 is formed with irrigation pores or apertures 27 (see **FIG. 7**) through which the fluid can exit from the interior of the balloon 80 to outside the balloon for cooling the tissue ablation site. While **FIG. 4** shows fluid exiting the balloon 80 as jet streams, it is understood that the fluid may exit the balloon with any desired flow rate and/or pressure, including a rate where the fluid is seeping out of the apertures 27.

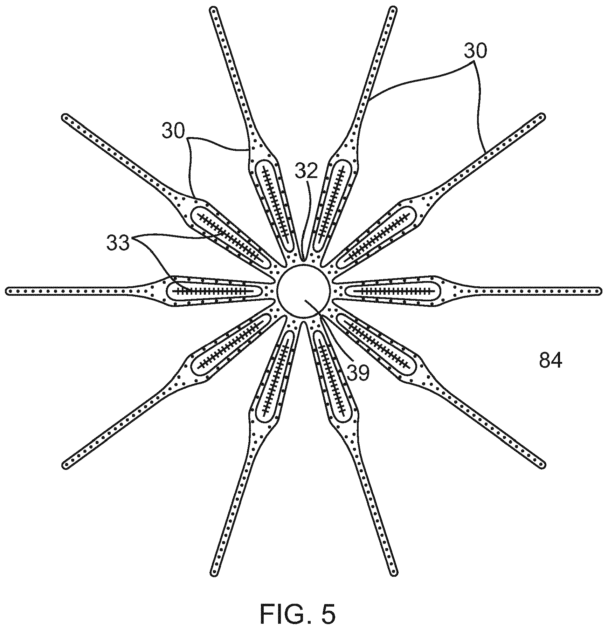

The membrane 26 supports and carries a combined electrode and temperature sensing member which is constructed as a multi-layer flexible circuit electrode assembly 84. The "flex circuit electrode assembly" 84 may have many different geometric configurations. In the illustrated embodiment, the flex circuit electrode assembly 84 has a plurality of radiating leaves or strips 30, as best seen in **FIG. 5****.** The leaves 30 are evenly distributed about the distal end 80D of the balloon 80. Each leaf has wider proximal portion that gradually tapers to a narrower distal portion.

With additional reference to **FIG. 3** and **FIG. 6A****,** each leaf 30 has a proximal tail 31 and is connected at its distal end to a hub 32 with a central opening 39 that is concentric with the distal end 80D of the balloon 80. The proximal tail 31 is tucked under and fastened to the catheter 24 by a proximal ring 28 mounted on the shaft 70. One or more contact electrodes 33 on each leaf come into galvanic contract with the ostium 11 during an ablation procedure, during which electrical current flows from the contact electrodes 33 to the ostium 11, as shown in **FIG. 4****.**

As shown in **FIG. 7****,** the flex circuit electrode assembly 84 includes a flexible and resilient sheet substrate 34, constructed of a suitable bio-compatible material, for example, polyimide. For each leaf 30, an outer surface 36 of the substrate 34 supports and carries a contact electrode 33 adapted for tissue contact with the ostium. The contact electrode 33 delivers RF energy to the ostium during ablation and/or is connected to a thermocouple junction for temperature/electropotential sensing of the ostium. In the illustrated embodiment, the contact electrode 33 has a longitudinally elongated portion 40 and a plurality of thin transversal linear portions or fingers 41 extending generally perpendicularly, evenly spaced between each other, from each lateral side of the elongated portion 40. Formed within the contact electrode 33 are one or more exclusion zones 47, each surrounding an irrigation aperture 35 formed in the substrate 34 which is in communication with a corresponding irrigation aperture 27 formed in the balloon membrane 26. Also formed in the contact electrode 33 are one or more conductive blind vias 48 which are conductive or metallic formations or substances that extend through through-holes (not shown) in the substrate 34 and are configured as electrical conduits connecting the contact electrode 33 and a wiring electrode 38 sandwiched between the substrate 34 and the balloon membrane. It is understood that "conductive" is used herein interchangeably with "metallic" in all relevant instances.

The wiring electrode 38 is generally configured as an elongated body similar in shape and size to the elongated portion 40 of the contact electrode 33. The wiring electrode 38 loosely resembles a "spine" and can also function as a spine in terms of providing a predetermined degree of longitudinal rigidity to each leaf 30 of the electrode assembly 84. The wiring electrode 38 is positioned such that each of the blind vias 48 is in conductive contact with both the contact electrode 33 and the wiring electrode 38. In the illustrated embodiment, the two electrodes 33 and 38 are in longitudinal alignment with each other, with all blind vias 48 in conductive contact with both electrodes 33 and 38.

The wiring electrode 38 is also formed with its exclusion zones 59 around the irrigation apertures 35 in the substrate 34. The wiring electrode 38 is further formed with at least one active solder pad portion 61. Attached, e.g., by a solder weld 63, to the active solder pad portion 61 are a wire pair, e.g., a constantan wire 51 and a copper wire 53. The copper wire 53 provides a lead wire to the wiring electrode 33, and the copper wire 53 and the constantan wire 51 provide a thermocouple whose junction is at solder weld 63. As illustrated, the wire pair 51/53 run between the membrane 26 and the substrate 34 and further proximally between the membrane 26 and the proximal tail 31 until the wire pair 51/53 enters the tubular shaft 70 via one or more through-holes 72 formed in the tubular shaft sidewall closer to the proximal ring 28, as shown in **FIG. 3** and **FIG. 6A****.**

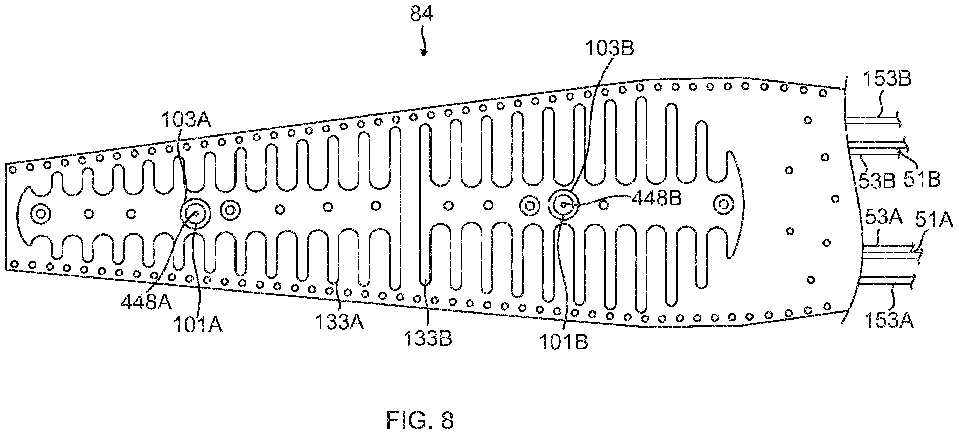

In some embodiments, as shown in FIG. 8, the flex circuit electrode assembly 84, may include split "island" contact microelectrodes 101A and 101B, physically and electrically isolated from a partially or fully surrounding contact electrode, such as "split" contact electrodes 133A and 133B, respectively. Corresponding split "island" wiring microelectrodes 103A and 103B are physically and electrically isolated from a partially or fully surrounding underlying wiring electrode 38 (see FIG. 7), which are also "split" wiring electrodes (not shown). Pairs of aligned contact microelectrodes 101A, 101B, and wiring microelectrodes 103A, 103B are conductively connected to each other by respective blind vias 448A, 448B. The microelectrodes 101A, 101B and 103A, 103B are configured for impedance, electrical signals, and/or temperature sensing independently of the electrodes 133A, 133B and 38. In a disclosed embodiment of FIG. 8, each of the split wiring electrodes has its own wire pair 51A/53A and 51B/53B, and each wiring microelectrode has its own wire (e.g., copper) 153A and 153B.

In other embodiments of the present disclosure, the balloon includes contact electrodes painted on the balloon membrane, such as with a conductive ink. In certain embodiments, a conductive material forming contact electrodes is applied by a micropen or positive displacement dispensing system, as understood by one of ordinary skill in the art. A micropen can dispense a controllable volume of paste per time, which enables control of thickness by varying print volume, paste concentration, and write speed. Such a system is disclosed in U.S. Patent No. 9,289,141, titled "Apparatus and Methods for the Measurement of Cardiac Output." Positive displacement dispensing technologies and direct-write deposition tools including aerosol jets and automated syringes are available under the mark MICROPEN by MicroPen Technologies and Ohmcraft, Inc., both of Honeoye Falls, N.Y. It is understood that the contact electrode 33 may assume any variety of patterns.

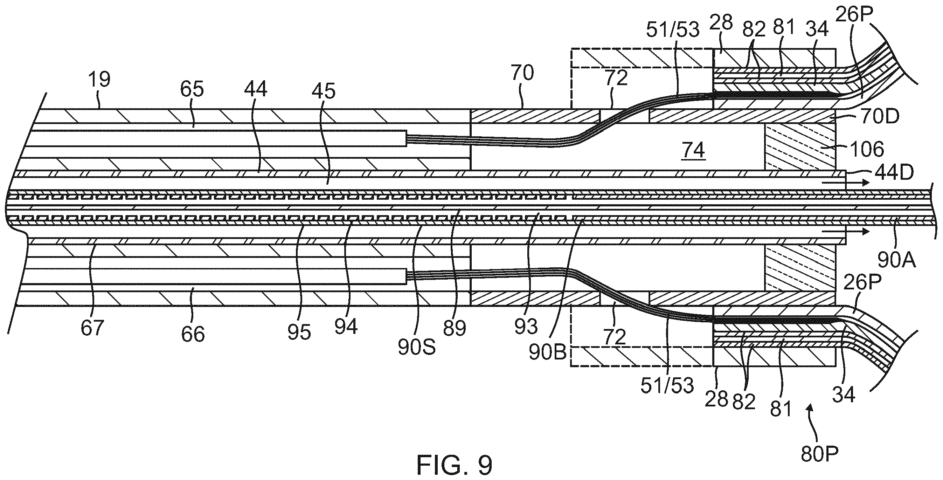

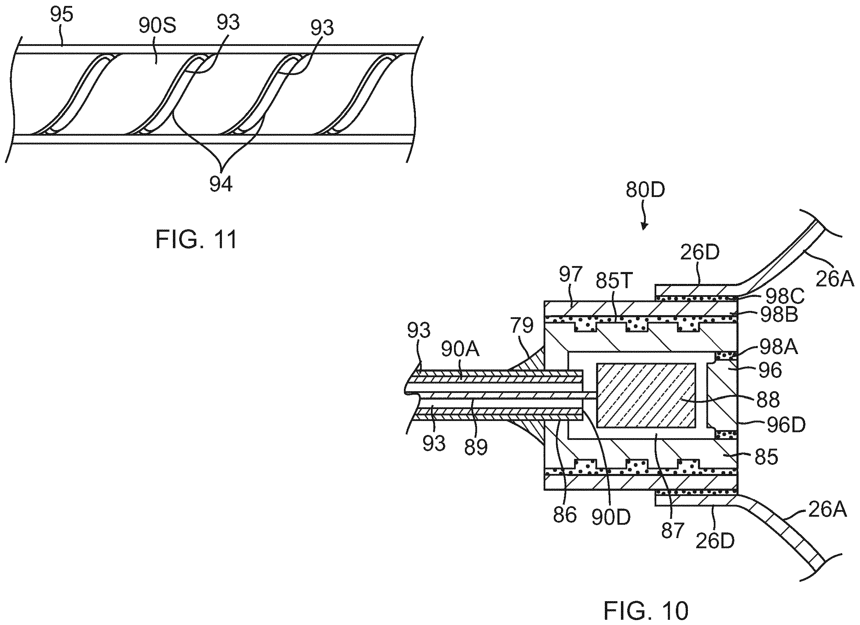

With reference to **FIG. 2A****,** the longitudinal and radial dimensions of the balloon 80 can be varied with longitudinal movement of an expander 90 relative to the shaft 70. The balloon 80 can adopt different configurations, including (1) a compressed configuration C (broken lines) where the expander 90 is drawn proximally to a proximal position relative to the shaft 70, (2) an elongated configuration E (broken lines) where the expander 90 is extended distally to a distal position relative to the shaft 70, and (3) a more neutral configuration N (solid lines) where the expander 90 is in between its distal and proximal positions. In some embodiments, as shown in **FIG. 9** and **FIG. 10****,** the expander 90 is configured as an elongated hollow tubing or rod with a lumen 93. The expander 90 has a distal end 90D at the distal end 80D of the balloon and can be described as having at least a distal portion 90A that spans the length of the balloon, and a proximal portion 90B that spans between the proximal end 80P of the balloon 80 and the control handle 16.

From the control handle, the proximal portion 90B extends through the central lumen (not shown) of the catheter body 17, the on-axis lumen 67 of the intermediate section 19 (see **FIG. 2B**), and the lumen 74 of the connector shaft 70 (see **FIG. 9****).** A segment 90S of the expander 90, e.g., at least the segment extending through the lumen 67 of the intermediate section 19, has one or more flexure slits for increased flexibility. In the illustrated embodiment of **FIG. 11****,** the portion 90S has a spiral slit 94 in its sidewall that coils along the length of the segment 90S. To seal the expander 90 at least along the segment 90S with the one or more flexure splits, a heat shrink sleeve 95 surrounds the expander.

Throughout the length of the catheter shaft, the proximal portion 90A of the expander 90 passes through a lumen 45 of an irrigation tubing 44 (see **FIG. 2B** and **FIG. 9**) which is longitudinally coextensive with the expander between the proximal end 80P of the balloon and into the control handle 16. The diameter of the irrigation tubing 44 is sized to provide a lumen 45 which accommodates the expander 90 and allows for irrigation fluid to pass through the irrigation tubing 44 and into the interior of the balloon 80 at the proximal end 80P of the balloon. Irrigation fluid delivered into the balloon can exit the balloon through the irrigation apertures 27 formed in the balloon membrane 26 and the irrigation apertures 35 formed in the flex circuit substrate 34 to cool surrounding tissue (see **FIG. 4**).

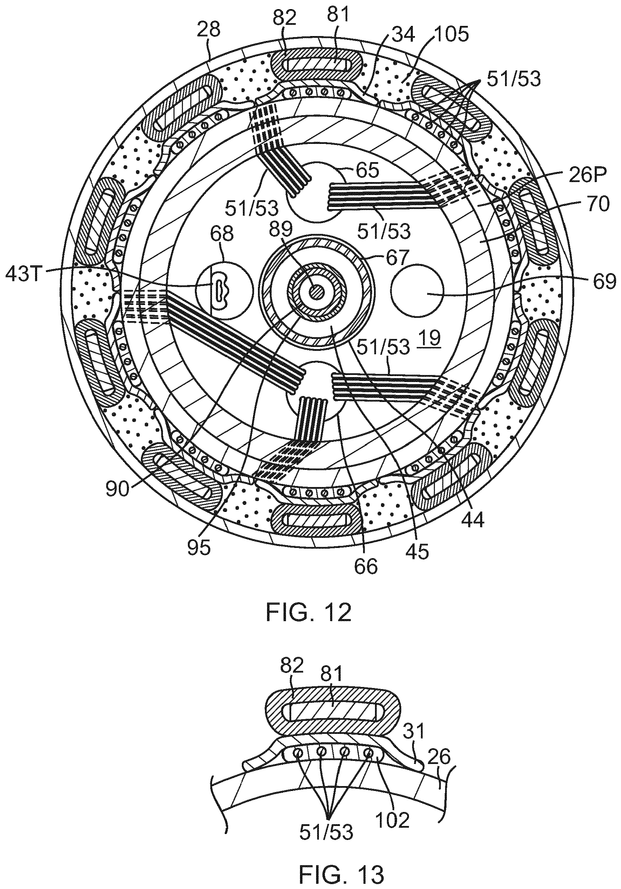

In the illustrated embodiment of **FIG. 9** and **FIG. 12****,** the proximal end 80P of the balloon includes an outer proximal ring 28 circumferentially surrounding a distal end 44D of the irrigation tubing 44. Sandwiched between the ring 28 and the distal end 44D are several components of the balloon 80, as described further below, which are affixed within the ring 28 with adhesive 105, e.g., epoxy. The proximal end 80P of the balloon includes an annular plug 106 filling the gap in the lumen 74 between the shaft 70 and the irrigation tubing 44. Adhesive (not shown) may be applied between an inner surface of the balloon membrane 26 and an outer surface of the shaft 70 to provide a fluid tight seal at the proximal end 80P. Adhesive (not shown) may also be applied between the plug 106 and an inner surface of the shaft 70 and/or an outer surface of the irrigation tubing 44 to provide a fluid tight seal at the proximal end 80P.

With the distal end of the irrigation tubing 44 terminating at the proximal end 80P of the balloon 80, the distal portion 90A of expander extending through an interior of the balloon 80, is without the irrigation tubing 44. In the illustrated embodiment of **FIG. 10****,** the distal end 80D of the balloon includes a sensor housing 85 having a hollow cylindrical body which has a passage 86 receiving the distal end 90D of the expander 90. For example, laser welding 79 secures the attachment and coupling of the distal end 90D and the housing 85. An interior 87 of the housing 85 houses an electromagnetic position sensor 88 whose cable 89 extends proximally through the lumen 93 of the expander 90 along the length of the catheter shaft and into the control handle 16. A distal end of the housing 85 includes a distal member 96 having a flat distal face 96D, that is affixed by adhesive 98A which also seals the interior 87 against fluid leaks. In some embodiments, the distal member 96 is configured as a distal tip electrode whose lead wire (not shown) may also pass proximally to the control handle via the distal passage 86 and through the lumen 93 of the expander 90. Optionally, lead wires for the various electrodes can be routed through lumen 93 and out of housing 85 at its distal end and into contact with the electrodes. The housing 85 may be constructed of any suitable material, including, for example, stainless steel, braided shafts, and the like.

In the disclosed embodiment, the housing 85 includes a cover 97 configured, e.g., as short tubing, circumferentially surrounding the housing body. An outer surface of the housing body may include a texture 85T with an uneven surface to better hold adhesive 98B affixing the cover 97 to the housing 85. Affixed to an outer radial surface of the cover 97 of the housing 85 by adhesive 98C is a distal end portion 26D of the balloon membrane 26 turned inwardly such that an outer surface 26A of the membrane 26 is affixed to the outer radial surface of the cover 97. Accordingly, the inward turn of the balloon membrane 26D and the flat distal face 96D of the distal member 96 advantageously provide the distal end 80D of the balloon with an atraumatic distal profile, as shown in **FIG. 3****,** which can contact tissue head-on without damaging tissue. With the balloon membrane distal end 26D affixed to the housing 85 and the housing 85 affixed to the distal end 90D of the expander, longitudinal movement of the expander 90 at its proximal end (either within the control handle 16, or proximal of the control handle) by a user can vary the configuration of the balloon, by elongating or compressing the balloon's longitudinal profile, as shown in **FIG. 2A****.** Moreover, the encased position sensor 88 is configured to generate electrical signals representative of the position of the distal end 80D.

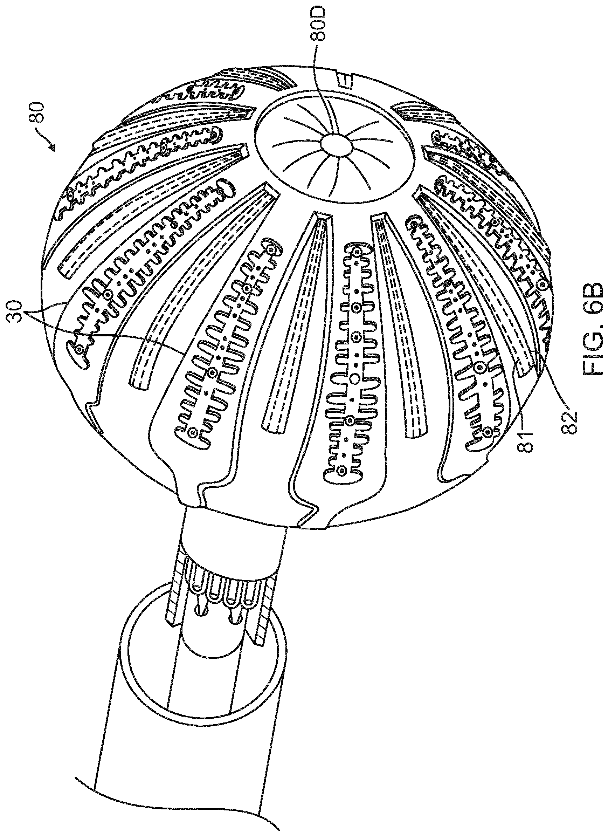

With reference to **FIGS. 6A** **and** **6B****,** the balloon 80 includes a plurality of elongated longitudinal supports or "spines" 81 extending radially from a proximal or distal end of the balloon 80 to a location on the outer surface of the balloon membrane 26 proximal to the distal end, or distal to the proximal end. That is, the ends of the spines fall around an equatorial portion of the balloon. The support spines 81 are made of a suitable material with shape-memory, for example, nitinol. The spines may have any suitable cross-sectional shape, e.g., rectangular or circular, and can be hollow, and are pre-shaped with a curvature to ensure that the balloon 80 assumes a generally spherical configuration when deployed from the distal end of the shaft 70 and especially when inflated with irrigation fluid. In some embodiments, each spine 81 is covered by a cover 82 configured, e.g., as a strip or a sleeve, that is affixed to an outer surface of the balloon membrane 26 and provides an interior passage through which the spine 81 extends. A distal end of the passage is sealed, e.g., by a plug of polyurethane 83. A proximal portion of each sleeve 82, along with a proximal portion of the respective spine 81, is tucked under and fastened to the balloon 80 by the proximal ring 28.

It is understood that the lengths of the sleeves 82 and the spines 81 may be different for different embodiments, as appropriate or desired. Likewise, the placement of the sleeves and the spines on the balloon 80 may be different for different embodiments, as appropriate or desired. In the illustrated embodiment of **FIG. 3** and **FIG. 6A****,** each sleeve 82 and each spine 81 have a length generally equal to the length of a tail 31. Moreover, each sleeve 82 is affixed to an outer surface of a respective tail 31, so that each spine 81 is generally coextensive with a respective tail 31, which in turn, cover lead wires 51, 53 from the flex circuit electrode assembly, as shown in **FIG. 13****.** The lead wires 51, 53 may be covered by a nonconductive protective cover to form a lead wire ribbon 102. Spines and sleeves may also lie along fold lines 76 of the balloon membrane in addition to or in lieu of the spines 81 and sleeves 82, as needed or desired. As such, these spines reinforce a proximal hemisphere of the balloon 80 so that the balloon 80 can better remain on axis relative to the shaft 70 when the balloon 80 contacts the ostium.

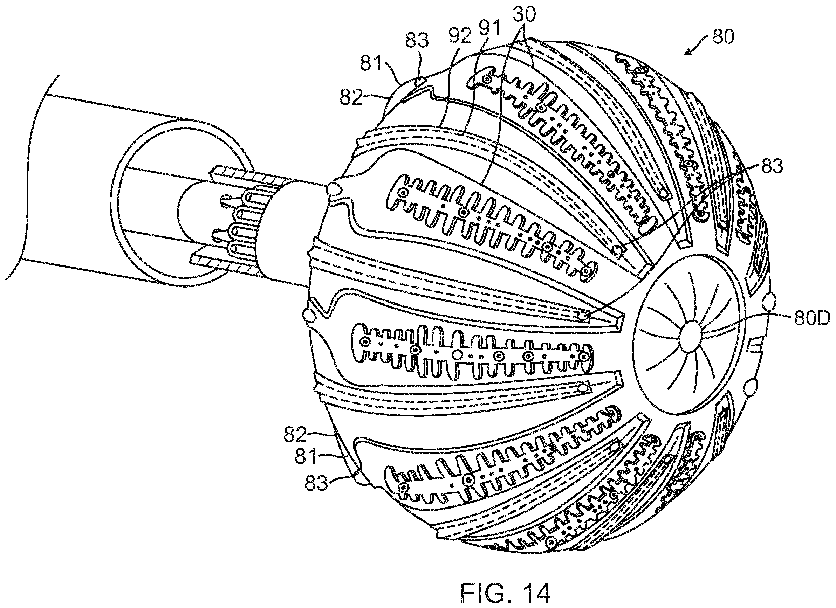

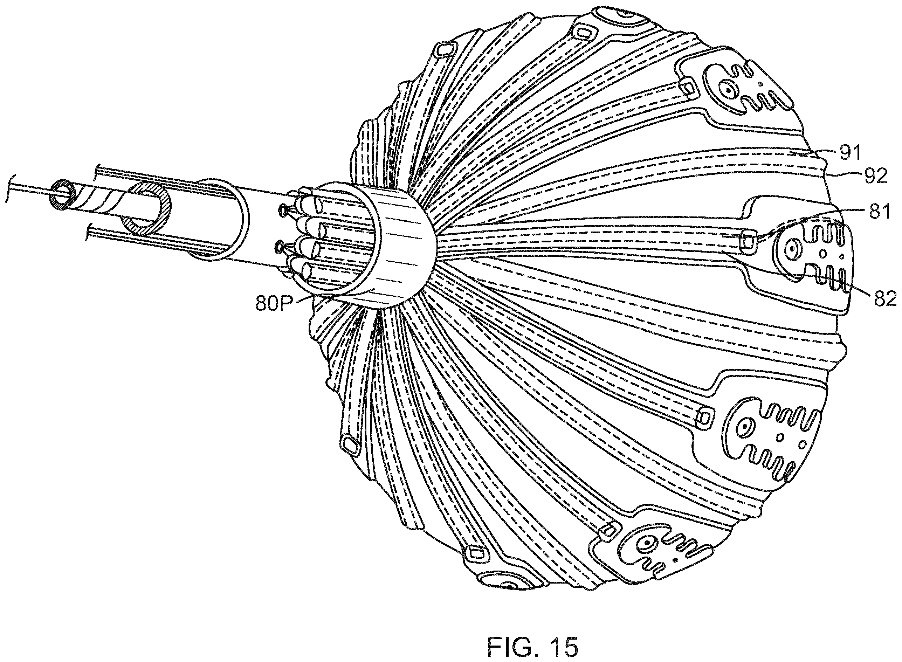

In another embodiment, as shown in **FIG. 14** and **FIG. 15****,** the balloon 80 includes spines 91 that extend the length of the balloon generally spanning both proximal and distal hemispheres of the balloon 80 between the distal and proximal ends 80D and 80P. Each spine extends through a cover 92, e.g., strips or sleeves, that is affixed to the outer surface of the balloon membrane 26 and provides an interior passage through which the spine 92 extends. A distal end of the passage is sealed, e.g., by a plug of polyurethane 83. The spines 91 in their covers 92 extend between the leaves 30 and the spines 81, e.g., lying on the fold lines 76. The spines 91 may be in addition to and/or in lieu of the spines 81, as appropriate or desired, in supporting the shape of the balloon.

The interior of the covers 82 and 92 may be shaped and sized to accommodate additional components, such as lead wires or cables, which would be protected and/or insulated from exposure to the patient's bodily fluids or irrigation fluid entering and exiting the interior of the balloon.

In some embodiments, the catheter includes a deflection puller wire 43 that extends through the central lumen of the catheter body 17, and the lumen 68 of intermediate section 19, the latter shown in **FIG. 2B**. A proximal end (not shown) of the puller wire is anchored in the control handle, and a distal end terminating in a T-bar 43T is anchored in a sidewall of the lumen 68 at or near a distal end of the multi-lumened intermediate section 19 (see **FIG. 12**). As understood in the art, a compression coil (not shown) surrounds the portion of the deflection puller wire extending through the catheter body 17, and has a distal end terminating generally at junction between the catheter body 17 and the intermediate section 19. The control handle includes a deflection mechanism (not shown) that acts on the puller wire to draw it proximally for deflecting the catheter.

As shown in **FIG. 9****,** the lead wires 51 and 53 leading from the flex circuit leaves 30 enter the lumen 74 of the connector shaft 70 via the one or more through-holes 72 situated at different radial locations around the connector shaft 70. Depending on factors, including, e.g., the plurality of leaves 30, the plurality of contact electrodes 33 and wiring electrodes 38, microelectrodes 101 and 103, the plurality of through-holes 72 varies, as desired or appropriate, to accommodate the plurality of lead wires 51 and 53. In any event, the lead wires 51 and 53 pass into the lumen 65 and/or the lumen 66 of the intermediate section 19, as shown in **FIG. 2B****,** and further proximally into the center lumen (not shown) of the catheter body 19. The holes 72 and the wires 51 and 53 may be protected and sealed by a suitable adhesive, e.g., epoxy. Moreover, the proximal ring 28 (as shown in broken lines in FIG. 9) may be sized to cover the holes and the wires, and sealed with a suitable adhesive.

## Claims

1. An electrophysiology catheter, comprising:
an elongated catheter shaft (70) having a first lumen (74);
a balloon (80) distal of the catheter shaft, the balloon having a distal end and a proximal end defining a longitudinal axis, the balloon including a membrane (26) and a contact electrode (33) supported on an outer surface of the membrane, the membrane defining an interior of the balloon;
an irrigation tubing (44) extending through the first lumen of the catheter shaft, the irrigation tubing having a second lumen, the irrigation tubing having a distal end terminating generally at the proximal end of the balloon; and
an elongated expander (90) having a first portion extending through the second lumen of the irrigation tubing, and a second portion extending through the proximal end of the balloon and into the interior of the balloon, the expander having a distal end coupled to the distal end of the balloon, the expander being longitudinally movable relative to the catheter shaft to move the distal end of the balloon in changing a configuration of the balloon,
wherein the balloon (80) further comprises a plurality of support spines (81) extending longitudinally along the outer surface of the membrane (26) of the balloon,
**characterised in that**
the support spines (81) are made of a material with shape-memory and are pre-shaped with a curvature to ensure that the balloon (80) assumes a generally spherical configuration.

2. The electrophysiology catheter of claim 1, wherein (i) at least one support spine (81) extends between the proximal end and distal end of the balloon (80), (ii) at least one support spine extends from the proximal end to a location on the outer surface of the membrane proximal of the distal end of the balloon, or (iii) at least one support spine extends from the distal end to a location on the outer surface of the membrane distal of the proximal end of the balloon.

3. The electrophysiology catheter of claim 1, wherein the balloon (80) further comprises a plurality of covers (82) affixed to the balloon membrane (26), and at least one cover covering the at least one support spine.

4. The electrophysiology catheter of claim 1, wherein the distal end of the balloon (80) has a flat distal face (96D).

5. The electrophysiology catheter of claim 4, wherein the distal end includes a housing (85) having the flat distal face, and an outer radial surface, wherein a distal end portion of the balloon membrane (26) is turned inwardly and affixed to the outer radial surface.

6. The electrophysiology catheter of claim 1, wherein the expander (90) has a section with a flexure slit.

7. The electrophysiology catheter of claim 6, wherein the flexure slit includes a spiral slit (94).

8. The electrophysiology catheter of claim 5, further comprising a position sensor (88) housed in the housing (85).

9. The electrophysiology catheter of claim 8, wherein the expander (90) includes a third lumen (93), and the position sensor (88) includes a cable (89) extending through the third lumen.

10. The electrophysiology catheter of claim 5, wherein the housing (85) includes a distal electrode (96) having the flat distal face (96D).

11. The electrophysiology catheter of claim 1, wherein the contact electrode (33) includes painted conductive ink.

## Patentansprüche

1. Elektrophysiologie-Katheter, umfassend:
einen länglichen Katheterschaft (70) mit einem ersten Lumen (74);
einen Ballon (80) distal vom Katheterschaft, wobei der Ballon ein distales Ende und ein proximales Ende aufweist, die eine Längsachse definieren, wobei der Ballon eine Membran (26) und eine auf einer Außenfläche der Membran getragene Kontaktelektrode (33) aufweist, wobei die Membran einen Innenraum des Ballons definiert;
einen Irrigationsschlauch (44), der sich durch das erste Lumen des Katheterschafts erstreckt, wobei der Irrigationsschlauch ein zweites Lumen aufweist, wobei der Irrigationsschlauch ein distales Ende aufweist, das im Allgemeinen am proximalen Ende des Ballons endet; und
einen länglichen Expander (90) mit einem ersten Abschnitt, der sich durch das zweite Lumen des Irrigationsschlauchs erstreckt, und einem zweiten Abschnitt, der sich durch das proximale Ende des Ballons und in den Innenraum des Ballons erstreckt, wobei der Expander ein distales Ende aufweist, das mit dem distalen Ende des Ballons gekoppelt ist, wobei der Expander bezüglich des Katheterschafts längs bewegbar ist, um das distale Ende des Ballons bei Veränderung einer Konfiguration des Ballons zu bewegen,
wobei der Ballon (80) ferner eine Vielzahl von Stützdornen (81) umfasst, die sich längs entlang der Außenfläche der Membran (26) des Ballons erstrecken,
**dadurch gekennzeichnet, dass**
die Stützdorne (81) aus einem Material mit Formgedächtnis gefertigt sind und mit einer Krümmung vorgeformt sind, um sicherzustellen, dass der Ballon (80) eine im Allgemeinen kugelförmige Konfiguration annimmt.

2. Elektrophysiologie-Katheter nach Anspruch 1, wobei (i) sich zumindest ein Stützdorn (81) zwischen dem proximalen Ende und dem distalen Ende des Ballons (80) erstreckt, (ii) sich zumindest ein Stützdorn vom proximalen Ende zu einer Stelle auf der Außenfläche der Membran proximal vom distalen Ende des Ballons erstreckt, oder (iii) sich zumindest ein Stützdorn vom distalen Ende zu einer Stelle auf der Außenfläche der Membran distal vom proximalen Ende des Ballons erstreckt.

3. Elektrophysiologie-Katheter nach Anspruch 1, wobei der Ballon (80) ferner eine Vielzahl von Abdeckungen (82) umfasst, die an der Ballonmembran (26) befestigt sind, und wobei zumindest eine Abdeckung den zumindest einen Stützdorn abdeckt.

4. Elektrophysiologie-Katheter nach Anspruch 1, wobei das distale Ende des Ballons (80) eine flache distale Fläche (96D) aufweist.

5. Elektrophysiologie-Katheter nach Anspruch 4, wobei das distale Ende ein Gehäuse (85) aufweist, das die flache distale Fläche und eine radiale Außenfläche aufweist, wobei ein distaler Endabschnitt der Ballonmembran (26) nach innen gewendet ist und an der radialen Außenfläche befestigt ist.

6. Elektrophysiologie-Katheter nach Anspruch 1, wobei der Expander (90) einen Abschnitt mit einem Biegungsschlitz aufweist.

7. Elektrophysiologie-Katheter nach Anspruch 6, wobei der Biegungsschlitz einen spiralförmigen Schlitz (94) aufweist.

8. Elektrophysiologie-Katheter nach Anspruch 5, ferner umfassend einen Positionssensor (88), der im Gehäuse (85) untergebracht ist.

9. Elektrophysiologie-Katheter nach Anspruch 8, wobei der Expander (90) ein drittes Lumen (93) aufweist und der Positionssensor (88) ein sich durch das dritte Lumen erstreckendes Kabel (89) aufweist.

10. Elektrophysiologie-Katheter nach Anspruch 5, wobei das Gehäuse (85) eine distale Elektrode (96) aufweist, die die flache distale Fläche (96D) aufweist.

11. Elektrophysiologie-Katheter nach Anspruch 1, wobei die Kontaktelektrode (33) lackierte leitfähige Tinte enthält.

## Revendications

1. Cathéter d'électrophysiologie, comprenant :
une tige de cathéter allongé (70) ayant une première lumière (74) ;
un ballonnet (80) distal de la tige de cathéter, le ballonnet ayant une extrémité distale et une extrémité proximale définissant un axe longitudinal, le ballonnet comprenant une membrane (26) et une électrode de contact (33) supportée sur une surface externe de la membrane, la membrane définissant un intérieur du ballonnet ;
un tuyau d'irrigation (44) s'étendant à travers la première lumière de la tige de cathéter, le tuyau d'irrigation ayant une deuxième lumière, le tuyau d'irrigation ayant une extrémité distale se terminant généralement au niveau de l'extrémité proximale du ballonnet ; et
un dispositif d'expansion allongé (90) ayant une première partie s'étendant à travers la seconde lumière du tuyau d'irrigation, et une seconde partie s'étendant à travers l'extrémité proximale du ballonnet et à l'intérieur du ballonnet, le dispositif d'expansion ayant une extrémité distale couplée à l'extrémité distale du ballonnet, le dispositif d'expansion étant mobile longitudinalement par rapport à la tige du cathéter pour déplacer l'extrémité distale du ballonnet lors du changement d'une configuration du ballonnet,
dans lequel le ballonnet (80) comprend en outre une pluralité de colonnes de support (81) s'étendant longitudinalement le long de la surface externe de la membrane (26) du ballonnet,
**caractérisé en ce que**
les colonnes de support (81) sont faites d'un matériau à mémoire de forme et sont préformées avec une courbure pour garantir que le ballonnet (80) adopte une configuration généralement sphérique.

2. Cathéter d'électrophysiologie selon la revendication 1, dans lequel (i) au moins une colonne de support (81) s'étend entre l'extrémité proximale et l'extrémité distale du ballonnet (80), (ii) au moins une colonne de support s'étend depuis l'extrémité proximale jusqu'à un emplacement sur la surface externe de la membrane proximale de l'extrémité distale du ballonnet, ou (iii) au moins une colonne de support s'étend depuis l'extrémité distale jusqu'à un emplacement sur la surface externe de la membrane distale de l'extrémité proximale du ballonnet.

3. Cathéter d'électrophysiologie selon la revendication 1, dans lequel le ballonnet (80) comprend en outre une pluralité de revêtements (82) fixés à la membrane de ballonnet (26), et au moins un revêtement recouvrant la ou les colonnes de support.

4. Cathéter d'électrophysiologie selon la revendication 1, dans lequel l'extrémité distale du ballonnet (80) comporte une face distale plate (96D).

5. Cathéter d'électrophysiologie selon la revendication 4, dans lequel l'extrémité distale comprend un boîtier (85) ayant la face distale plate, et une surface radiale externe, dans lequel une partie d'extrémité distale de la membrane de ballonnet (26) est tournée vers l'intérieur et fixée à la surface radiale externe.

6. Cathéter d'électrophysiologie selon la revendication 1, dans lequel le dispositif d'expansion (90) comporte une section ayant une fente de flexion.

7. Cathéter d'électrophysiologie selon la revendication 6, dans lequel la fente de flexion comprend une fente en spirale (94).

8. Cathéter d'électrophysiologie selon la revendication 5, comprenant en outre un capteur de position (88) logé dans le boîtier (85).

9. Cathéter d'électrophysiologie selon la revendication 8, dans lequel le dispositif d'expansion (90) comprend une troisième lumière (93), et le capteur de position (88) comprend un câble (89) s'étendant à travers la troisième lumière.

10. Cathéter d'électrophysiologie selon la revendication 5, dans lequel le boîtier (85) comprend une électrode distale (96) ayant la face distale plate (96D).

11. Cathéter d'électrophysiologie selon la revendication 1, dans lequel l'électrode de contact (33) comprend une encre conductrice peinte.
